(19) Europäisches Patentamt
European Patent Office
Office européen des brevets

(11) Publication number: **0 000 950**
**B1**

(12) **EUROPEAN PATENT SPECIFICATION**

(45) Date of publication of patent specification: **01.02.84**

(51) Int. Cl.³: **C 07 D 233/58**
//A61K31/415

(21) Application number: **78100753.9**

(22) Date of filing: **25.08.78**

(54) **Imidazole derivatives and salts thereof and their synthesis.**

(30) Priority: **26.08.77 GB 3591277**
**01.02.78 GB 398378**
**08.08.78 GB 3253678**
**22.08.78 GB 3408978**

(43) Date of publication of application:
**07.03.79 Bulletin 79/5**

(45) Publication of the grant of the patent:
**01.02.84 Bulletin 84/5**

(84) Designated Contracting States:
**BE CH DE FR GB LU NL SE**

(56) References cited:
**DE - A - 2 533 211**

**BIOCHEMICAL AND BIOPHYSICAL RESEARCH COMMUNICATIONS, Vol. 80, No. 1, 1978, pp. 236-242**

**The file contains technical information submitted after the application was filed and not included in this specification**

(73) Proprietor: **THE WELLCOME FOUNDATION LIMITED**
**183-193 Euston Road**
**London NW1 2BP (GB)**

(72) Inventor: **Thorogood, Peter Brian**
**114 Venner Road**
**Sydenham London, S.E. 26 (GB)**

(74) Representative: **Berg, Wilhelm, Dr. et al,**
**Dr. Berg, Dipl.-Ing. Stapf, Dipl.-Ing. Schwabe, Dr. Dr. Sandmair Patentanwälte Postfach 860245**
**D-8000 München 86 (DE)**

**EP 0 000 950 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Imidazole derivatives and salts thereof, and their synthesis

The present invention relates to imidazole derivatives and salts thereof, and their synthesis.

Thromboxane $A_2$ ($TXA_2$), a potent stimulator of blood platelet aggregation, is produced, in platelets, from the prostaglandin endoperoxides $PGG_2$ and $PGH_2$. Prostacyclin ($PGI_2$), which has potent anti-aggregatory activity, is also produced (in bloo dvessel walls) from $PGG_2$ and $PGH_2$ and it has been suggested that a balance between the production of $TXA_2$ and $PGI_2$ is the controlling factor in thrombus formation. It would, in consequence, be desired in the treatment and prophylaxis of thrombo-embolic disorders to be able to selectively inhibit $TXA_2$ synthetase, thereby favouring the production of the anti-aggregatory agent $PGI_2$.

Imidazole and 1-methylimidazole are known to provide some degree of inhibition of the enzymic conversion of the endoperoxides ($PGG_2$ and $PGH_2$) to thromboxane $A_2$ by platelet microsomes (Moncada et al., Prostaglandins, 13/4, 611—618, 1977). Certain 1-($C_{10-19}$) hydrocarbylimidazoles having been described as being capable of lowering serum cholesterol levels (U.K. Patent No. 1 364 312; Biochem. Pharmacol. 24, 1902—1903, 1975).

Also, 1-cyclohexylmethyl-imidazole is disclosed in Wiss. Z. Univ. Halle, Math-Nat. XI/5, 1962, pages 603—612 although no physiological activity is ascribed therein to the compound.

The inhibitory potency of several derivatives of imidazole and 1-substituted imidazoles, such as 1-methylimidazole on thromboxane synthetase is known from Biochemical and Biophysical Research Communications, Volume 80 No. 1, 1978, 236—242. The authors of this article report that the inhibitory potency of 1-substituted imidazoles is increased with increasing chain length and states that 1-nonyl imidazole and 1-(2-isopropylphenyl) imidazole were found to possess the highest potency. The best activities are obtained with compounds wherein the 1-substituent on the imidazole ring is a straight chain alkyl group and the reference gives no information as to the 1-substituted imidazoles carrying a cycloalkyl or cycloalkenyl group.

We have now discovered that $TXA_2$ synthetase can with a surprisingly high and selective effect be inhibited by 1-substituted imidazoles of formula (I) as herein defined and pharmaceutically acceptable acid addition salts thereof. The compounds of formula I and their pharmaceutically acceptable acid addition salts are herein referred to as the "active compounds".

The compounds of formula I are novel and of formula:

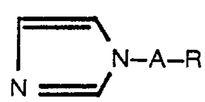

(I)

in which A is a straight or branched, saturated or unsaturated acyclic hydrocarbon radical of from 1 to 3 carbon atoms and R is a cycloalkyl or cycloalkenyl radical of from 4 to 9, preferably from 5 to 8 carbon atoms, optionally substituted by one or more alkyl radicals each containing from 1 to 4 carbon atoms with the proviso that when A is a methylene radical R is not unsubstituted cyclohexyl.

In formula (I) examples of cycloalkyl radicals are cyclobutyl, cyclopentyl, cyclohexyl, cycloheptyl, and cyclooctyl; cycloalkenyl radicals include cyclohex-3-enyl, cyclopentenyl, 1,4-cyclohexadienyl and cyclohept-2-enyl.

A valuable class of compounds of formula (I) are those in which R is cyclohexyl, cycloheptyl, cyclooctyl or cycloalkenyl of 6 to 8 carbon atoms and A is $-CH_2-$ or $-(CH_2)_2-$.

Compounds of formula (I) may also be used as pharmaceutically acceptable acid addition salts thereof.

Preferred compounds include:

1-Cyclooctylmethylimidazole
1-Cyclohex-3-enylmethylimidazole
1-Cyclohexylethylimidazole
1-Cycloheptylmethylimidazole

1-Cyclopentylmethylimidazole
1-(4-Methylcyclohexylmethyl)imidazole
1-Cyclobutylmethylimidazole
1-Cyclooctylvinylimidazole
1-(1-Cyclooctylethyl)imidazole
1-(2-Cyclooctylethyl)imidazole
1-(3-Cyclooctylpropyl)imidazole

1-(Cyclohept-2-enylmethyl)imidazole
1-Cyclononylmethylimidazole

and pharmaceutically acceptable acid addition salts thereof.

Especially preferred compounds include

1-Cyclohex-3-enylmethylimidazole,
1-Cycloheptylmethylimidazole,
1-Cyclooctylmethylimidazole and
1-Cyclohexylethylimidazole

and pharmaceutically acceptable acid addition salts thereof.

In contrast to imidazole and 1-methylimidazole the compounds of formula (I) are more potent inhibitors of $TXA_2$ synthetase. Many of the compounds (for example in (I) A is $—CH_2—$ or $—(CH_2)_2—$) are also more selective in their action in not inhibiting other prostaglandin-generating enzymes such as cyclo-oxygenase. The compounds of formula (I) also do not produce the side-effects found with imidazole upon *in vivo* administration. The compounds of formula (I) are further capable of inhibiting platelet aggregation *in vivo* and also are capable of disaggregating platelet clumps. The compounds 1 cyclooctylmethylimidazole, 1-cyclohex-3-enylmethylimidazole and 1-cyclohexylethylimidazole and their pharmaceutically acceptable salts especially displaying these properties.

Imidazoles of formula (I) and pharmaceutically acceptable acid addition salts thereof may be made by any method known in the art for the synthesis of compounds of analogous structure. In general these methods comprise linking the imidazole ring to the remainder of the molecule; converting a precursor molecule by elimination of a functional group from the imidazole ring; and formationn of the desired compound from a corresponding imidazoline, pyrazole or unsaturated analogue.

A most convenient method of synthesis involves the reaction of imidazole (formula II) or a salt thereof with an alkylating agent of formula (III):

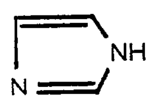

(II)

Z–A–R

(III)

wherein R and A are as defined in formula (I) and Z is a leaving group. This reaction is well established in the literature, and the leaving group may be chosen from a variety of substituents but especially halo, preferably chloro or bromo, or from p-toluenesulphonyloxy but other arylsulphonyloxy, alkanesulphonyloxy or aralkylsulphonyloxy radicals may be used. The reaction is preferably performed in the presence of an acid acceptor, for example an alkali metal alkoxide such as sodium methoxide or potassium tertiary butoxide in the presence of an corresponding alkanol. The leaving group Z may itself be formed *in situ* from the corresponding alkanol (Z = OH) by reaction with a hydrohalogenic acid (e.g. hydrochloric acid or a Lewis acid such as aluminium chloride: see Japanese Patent Kokai No. 131577/77) and the resulting agent of formula (III) reacting directly with imidazole without prior isolation. Alternatively an alkanol (Z = OH) or a derivative thereof (e.g. Z = R—A—O—) may be reacted directly with imidazole (II) by heating in the presence of a dehydrating agent such as phosphoric acid, or a phosphate (see Japanese Patent Publication No. 5 1105 060), sulphuric acid or sulphates (see Japanese Patent Publication No. 5 1105 061).

Among precursor molecules which may be converted to a compound of formula (I) or a pharmaceutically acceptable acid addition salt thereof, are substituted imidazole derivatives of formula (IV) or addition salts thereof

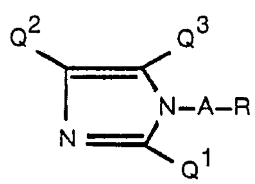

(IV)

wherein A and R are as defined in formula (I), and $Q^1$, $Q^2$ and $Q^3$ are the same or different, at least one being selected from thio (—SH), alkylthio (—S-alkyl wherein alkyl has 1 to 4 carbon atoms) and halo preferably chloro or bromo, the other being a radical having the same function or is hydrogen.

The reaction conditions are chosen according to the nature of the radicals $Q^1$, $Q^2$ and $Q^3$. Desulphurisation may be performed by oxidative or reductive procedures using for example nitric acid or Raney nickel; and reductive dehalogenation by the use of zinc and acetic acid or Raney nickel or other reagents known in the literature.

Another class of precursor molecules include carboxyimidazoles or derivatives thereof of formula (VI):

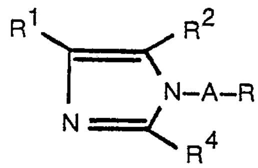

(VI)

wherein A and R are as defined in formula (I), at least one of $R^1$, $R^2$ and $R^4$ is carboxyl or a derivative thereof (for example an ester such as an alkyl ester, an acid halide such as the chloride, or the nitrile) and the other(s) is hydrogen or carboxyl or a derivative as described. The compounds of formula (VI) may be converted into the imidazoles of formula (I) by any suitable decarboxylation conditions which may simply comprise heating the compounds with or without a catalyst such as copper.

The imidazoles of formula (I) may also be made from a compound of formula (VII):

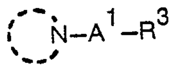 wherein 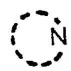

(VII)

is 1-imidazole or 1-pyrazole, $A^1$ is a straight or branched saturated or unsaturated acyclic hydrocarbon radical, and $R^3$ is a cycloalkyl or cycloalkenyl radical of from 4 to 9 carbon atoms optionally substituted by alkyl as defined in formula (I), provided that at least one of

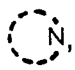

$A^1$ and $R^3$ is other than 1-imidazole, a saturated acyclic hydrocarbon and an optionally substituted cycloalkyl group respectively as defined in formula (I). Thus an imidazoline (VIII):

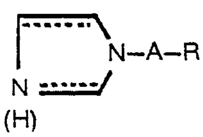

(VIII)

wherein one of ---- represents an extra bond, A and R are defined in formula (I) may be dehydrogenated to the corresponding imidazole in the presence of a catalyst for example by heating to 250°C in the presence of palladium, nickel or platinum under pressure, or by heating with a dehydrogenating agent such as selenium or copper oxide. The 1-pyrazole compounds (VII) may be treated with ultraviolet irradiation, optionally under an inert atmosphere (e.g. argon) in for example 1,2-dimethoxyethane at room or elevated temperatures (see for example "Ring Transformations of Heterocycles" edited van der Plas, Academic Press, 1973 at page 261). The unsaturated imidazoles of formula (I) (in formula (VII), $A^1$ and/or $R^3$ are unsaturated) may be reduced to the corresponding saturated compounds with a noble metal catalyst, for example platinum or palladium in an alkanol.

The intermediates for use in the above described reactions may also be made by conventional methods known in the art. Thus the 1-pyrazole and 1-imidazoline intermediates (formula (VII) may be prepared by alkylation of pyrazole and imidazoline in an analogos manner to that described above for preparation of the corresponding imidazoles. The intermediates of formula (III) may be made in known manner preferably by halogenation of the corresponding alcohols (formula (III), Z = —OH) where in such compounds R is cycloalkenyl, the alcohol is conveniently prepared by the Prins reaction from the cycloalkene and paraformaldehyde (Bull. Chem. Soc. Japan 46/8, 2512—5, 1973). The substituted imidazole intermediates of formula (IV) may be made in known manner, for example see "Imidazole and its derivatives" Part I, Ed. K. Hofmann, Interscience Publishers Inc. New York, 1973. For example the 2-thioimidazoles of formula (IV) may be made by cyclisation of an acetal of formula (IX):

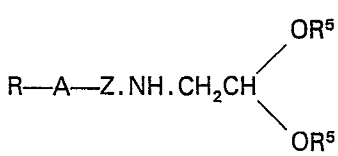

(IX)

with thiocyanate, wherein $R^5$ is alkyl.

The pharmaceutically acceptable addition salts of the compounds of formula (I) may be prepared by any method known in the art. In particular they may be prepared by treating the parent imidazole with the appropriate acid.

Examples of the addition salts of the compounds of formula (I) include those salts derived from the following acids: oxalic, hydrochloric, hydrobromic, sulphuric, nitric, perchloric, fumaric, maleic, phosphoric, glycolic, lactic, salicyclic, succinic, toluene-*p*-sulphonic, tartaric, acetic, citric, methanesulphonic, formic, benzoic, malonic, naphthalene-2-sulphonic and benzenesulphonic.

The imidazoles of formula (I) may be used in conjunction with a phosphodiesterase inhibitor, which provides a further, synergistic increase in effect, as it acts against platelet aggregation by a different pathway.

Suitable phosphodiesterase inhibitors for use in potentiating the anti-aggregatory effects of the active compounds include as such or as pharmaceutically acceptable salts:—

(a) Xanthine derivatives such as:—
Theophylline(3,7-dihydro-1,3-dimethyl-1*H*-purine-2,6-dione), and salts thereof.
3-Isobutyl-1-methyl-xanthine;
Caffeine (3,7-dihydro-1,3,7-trimethyl-1*H*-purine-2,6-dione) and salts thereof; and
Aminophylline (adduct of Theophylline and 1,2-ethanediamine (2:1)).

(b) Isoquinoline derivatives, for example:—
Papaverine(1-[(3,4-dimethoxyphenyl)methyl]-6,7-dimethoxyisoquinoline) and salts thereof; and
6,7-Diethoxy-1-(4,5-diethoxybenzyl)isoquinoline or its salts e.g. its hydrochloride;

(c) Derivatives of pyrimido(5,4-d)pyrimidine, for example:—
Dipyridamole(2,2′,2″,2‴-(4,8-dipiperidinopyrimido[5,4-d]pyrimidin-2,6-diyldinitrilo)tetraethanol) and its salts;
2,2′,2″,2‴-[[4-(1-piperidinyl)pyrimido[5,4-d]pyrimidin-2,6-diyl]dinitrilo]tetrakisethanol and its salts; and
2,4,6-tri-4-morpholinylpyrimido[5,4-d]pyrimidine and its salts.

(d) Derivatives of thieno[3,2-d]pyrimidine, for example:—
N-[4-(4-morpholinyl)thieno]3,2-d[pyrimidin-2-yl]-1,2-ethanediamine.

(e) Derivatives of pyrazolo[3′,4′:2,3]pyrido[4,5-b][1,5]benzodiazepin-6-(3*H*)-one, for example:—
3-Ethyl-7,12-dihydro-7,12-dimethylpyrazolo[4′,3′:5,6]pyrido[4,3-b]-[1,5]benzodiazepin-6-(3*H*)-one;
3-Ethyl-7,12-dihydro-9-methoxy-7,12-dimethylpyrazolo[3′,4′:2,3]pyrido[4,5-b][1,5]benzodiazepin-6-(3*H*)-one; and
10-Chloro-3-ethyl-7,12-dimethyl-7,12-dihydropyrazolo[4′,3′:5,6]pyrido[4,3-b][1,5]benzodiazepin-6-(3*H*)-one.

(f) Derivatives of 1*H*- or 2*H*-pyrazolo[3,4-b]pyridine, for example:—
4-(Butylamino)-1-ethyl-1*H*-pyrazolo[3,4-b]pyridine-5-carboxylic acid ethyl ester;
4-(Butylamino)-1*H*-pyrazolo[3,4-b]pyridine-6-carboxylic acid ethyl ester;
4-Chloro-1-ethyl-3-methyl-1*H*-pyrazolo[3,4-b]pyridine-5-acetonitrile;
1-Ethyl-4-(isopropylidenehydrazino)-3-methyl-1*H*-pyrazolo[3,4-b]pyridine-5-carboxylic acid ethyl ester or its salts such as its hydrochloride hemihydrate; and
2-Methyl-6-phenyl-4-(1-piperidinyl)-2*H*-pyrazolo[3,4-b]pyridine or its salts e.g. its hydrochloride.

(g) Derivatives of 5*H*-furo-[3,4-e]pyrazolo[3,4-b]pyridine-5-one, for example:—
4-(Butylamino)-1-ethyl-1,7-dihydro-7-hydroxy-5*H*-furo-[3,4-e]pyrazolo[3,4-b]pyridine-5-one; and

(h) Derivatives of 1(2*H*)-naphthalenone, for example:—
2[(Dimethylamino)methyl]-3,4-dihydro-7-methoxy-1(2*H*)-naphthalenone or its salts e.g. its 1:1 hydrochloride.

The active compounds are particularly useful in the treatment and/or prophylaxis of thrombo-embolic disorders in mammals, including man. It is to be understood that the term "thrombo-embolic disorders" includes those disorders whose etiology is associated with platelet aggregation.

The active compounds are useful wherever it is desired to inhibit platelet aggregation and/or to reduce the adhesive character of platelets, and consequently to treat or prevent the formation of thrombi in mammals, including man. For example, the compounds are useful in the treatment and prevention of myocardial infarcts cerebro-vascular thrombosis and ischaemic peripheral vascular disease; to treat and prevent post-operative thrombosis; and to promote patency of vascular grafts following surgery.

The active compounds are also useful as an addition to blood, blod products, blood substitutes, and other fluids which are used in artificial extra-corporeal circulation and perfusion of isolated body

portions, e.g., limbs and organs, whether attached to the oridinal body, detached and being preserved or prepared for transplant, or attached to a new body. It may also be used in laboratory animals, e.g. cats, dogs, rabbits, monkeys and rats, for these purposes in order to develop new methods and techniques for organ and limb transplants.

The active compounds also exhibit some vasodilatory action on blood vessels and therefore have a utility as anti-hypertensives for the treatment of high blood pressure in mammals, including man.

The amount of active compound required for therapeutic or prophylactic effect will vary with the route of administration, and the nature of the condition under treatment. In general a suitable dose for a mammal, including man, of active compound will lie in the range of 0.1 to 300 mg per kg body weight, particularly from 0.5 to 10 mg per kg body weight, for example 2 mg per kg. A suitable single oral dose for an adult human lies within the range of 50 to 600 mg, for example 150 mg given say three times a day.

While it is possible for the active compounds to be administered as the raw chemical it is preferable to present them as a pharmaceutical formulation. The formulations, both for veterinary and for human medical use, of the present invention comprise an active compound as above defined, together with one or more acceptable carriers therefor and optionally other therapeutic ingredients. The carrier(s) must be 'acceptable' in the sense of being compatible with the other ingredients of the formulation and not deleterious to the recipient thereof. Unit doses of a formulation may contain between 60 mg and 1.5 g of an active compound.

The formulations include those suitable for oral, rectal, vaginal or parenteral (including subcutaneous, intramuscular and intravanous) administration. Preferred formulations include tablets, capsules and injectable suspensions or solutions.

The formulations may conveniently be presented in unit dosage form and may be prepared by any of the methods well known in the art of pharmacy. All methods include the step of bringing into association the active compound (in the form of the base or a pharmaceutically acceptable acid addition salt) with the carrier which constitutes one or more accessory ingredients. In general the formulations are prepared by uniformly intimately bringing into association the active compound with liquid carriers or finely divided solid carriers or both, and then if necessary, shaping the product into the desired formulation.

It will be appreciated from the foregoing that the present invention provides the following features:—

(a) Novel compounds of formula (I), and pharmaceutically acceptable addition salts thereof.

(b) Methods of preparing imidazoles of formula (I) and pharmaceutically acceptable acid addition salts thereof.

The following Examples are provided by way of an illustration of the present invention and should in no way be construed as constituting a limitation thereof. All temperatures are given in degrees Celsius.

Example 1

*1-Cyclooctylmethylimidazole*

Imidazole (2.0 g, 0.03 mol) was added to a solution of sodium (0.7 g, 0.03 mol) in dry ethanol (50 ml). The mixture was stirred and heated to boiling when bromomethylcyclooctane (5.5 g, 0.027 mol) was added dropwise. Following the addition, the reaction mixture was stirred and boiled for 15 h.

After cooling, the reaction mixture was filtered, and the filtrate concentrated under reduced pressure. The residue was dissolved in 2*M*-hydrochloric acid (100 ml) and the solution washed with ether (25 ml). The aqueous layer was basified with 10*M*-sodium hydroxide solution and then extracted with chloroform (3 × 50 ml). The chloroform extracts were combined and dried ($MgSO_4$). Evaporation of the chloroform gave an oil which was purified using a silica gel column and elution with ethyl acetate/methanol (9:1). The product was further purified by distillation, b.p. 120—122°/(0.2 mmHg) 0.2666 mbar.

Example 2

*Preparation of 1-cyclopentylmethylimidazole*

Imidazole (6.8 g, 0.1 mol) was added to a solution of sodium (2.3 g, 0.1 mol) in dry ethanol (100 ml). This solution was stirred and heated to reflux when bromomethylcyclopentane (16.3 g, 0.1 mol) was added dropwise. Following the addition, the mixture was stirred and heated under reflux for 16 h.

After cooling, the reaction mixture was filtered and the filtrate concentrated under reduced pressure. The residue was dissolved in 2*M*-hydrochloric acid (150 ml) and the solution washed with ether. The aqueous solution was basified with 10*M*-sodium hydroxide solution, and the product extracted with chloroform (3 × 50 ml). The extracts were combined, dried ($MgSO_4$), and the solution concentrated to afford a yellow oil.

The oil was purified using a silica gel column and elution with ethyl acetate/methanol (9:1). The product fractions were combined and concentrated under reduced pressure to afford 1-cyclopentylmethylimidazole (1.9 g), which was further purified by distillation, b.p. 68—69°/(0.125 mmHg) 0.1667 mbar.

Example 3

*Preparation of 1-(3-cyclopentylpropyl)imidazole*

Imidazole (1.0 g, 0.0147 mol) was added to a solution of sodium (0.34 g, 0.0148 mol) in dry ethanol (30 ml). This solution was stirred and heated to boiling when 3-bromopropylcyclopentane (2.94 g, 0.0154 mol) was added dropwise. Following the addition, the reaction mixture was stirred and boiled for 20 h.

After cooling, the mixture was filtered and the filtrate concentrated under reduced pressure. The residue was dissolved in 2*M*-hydrochloric acid (50 ml) and the solution washed with ether (25 ml). The acid solution was then basified with 10*M*-sodium hydroxide solution, and the product extracted with chloroform (3 x 25 ml). The combined extracts were dried ($MgSO_4$) and concentrated under reduced pressure to afford a yellow oil (2.1 g).

The oil was purified by column chromatography (silica gel) using ethyl acetate/methanol (9:1) as eluent. The product fractions were combined and concentrated to afford 1-(3-cyclopentylpropyl)-imidazole which was further purified by distillation, b.p. 89—90°/(0.1 mmHg) 0.1333 mbar.

Example 4

*Preparation of 1-(cycloheptylmethyl)imidazole*

Bromomethylcycloheptane (5.3 g, 0.0278 mol) was added dropwise to a stirred solution of potassium *t*-butoxide (3.1 g, 0.0277 mol) and imidazole (1.9 g, 0.0279 mol) in dry *n*-butanol (50 ml) maintained at 100° and under dry nitrogen. After the addition (~20 mins) the temperature of the reaction mixture was raised to boiling. The reaction mixture was then stirred and boiled for 7 h and then cooled.

The mixture was filtered, and the *n*-butanol was removed under reduced pressure to give a pale yellow oil. The oil was dissolved in 2*M*-hydrochloric acid (100 ml) and the acid solution was washed with ether (100 ml) and then basified with 10*M*-sodium hydroxide solution and the resulting suspension was extracted with chloroform (3 x 50 ml). The chloroform extracts were combined, dried ($MgSO_4$), and concentrated under reduced pressure to give a pale yellow oil.

The oil was purified using a silica gel column and elution with ethyl acetate/methanol (9:1). Concentration of the fractions containing 1-(cycloheptylmethyl)imidazole gave a pale yellow oil which was further purified by distillation, b.p. 92—94°/(0.1 mmHg) 0.1333 mbar.

Example 5

*Preparation of 1-(2-cyclooctenylmethyl)imidazole*

(a) Preparation of 2-cyclooctene-1-methanol using the Prins reaction (Uchida et al., Bull. Chem. Soc., Japan, 1973, *46,* 2512)

Cyclooctene (69.0 g, 0.63 mol) was added dropwise, to a stirred suspension of paraformaldehyde (24.0 g) in 98% formic acid (100 ml). After the addition, the reaction mixture was stirred and heated under reflux for 2 h. Water (100 ml) was then added, and the aqueous solution was extracted with ether (50 ml). The ether solution was washed with saturated sodium bicarbonate solution (5 x 50 ml), with water (2 x 50 ml), and then dried ($MgSO_4$). Concentration under reduced pressure afforded a brown oil which was purified by distillation, the fraction b.p. 80—100°/(24 mmHg) 32 mbar being retained.

A portion of the aforsaid oil (10 g) was treated with Claisen's alkali [potassium hydroxide (10 g), methanol (31.2 ml) and water (8 ml)], and the reaction mixture was then boiled for 2 h.

After cooling, the mixture was poured onto iced-water (50 ml) and extracted with ether (3 x 50 ml). The ether extracts were combined and dried ($MgSO_4$). Concentration of the solution under reduced pressure afforded an oil which was distilled, to afford 2-cyclooctene-1-methanol, b.p. 128—130°/(23 mm hg) 30.7 mbar.

(b) Preparation of 2-cyclooctene-1-bromomethane

A solution of phosphorus tribromide (1.02 ml, 0,0105 mol) in petroleum ether (b.p. 40—60°, 5 ml) was added dropwise to a stirred solution of 2-cyclooctene-1-methanol (2.8 g, 0.02 mol) and dry pyridine (0.104 g, 0.0013 mol) in petroleum ether (b.p. 40—60°; 15 ml) at −10°. After the addition, the reaction mixture was set aside at ambient temperature for 48 h.

The reaction mixture was treated with water (50 ml) and the organic layer separated. The aqueous solution was extracted with petroleum ether (b.p. 40—60°, 3 x 25 ml) and the organic layer and petroleum ether extracts combined, washed with 2*M*-sodium hydroxide solution (25 ml), and with water (25 ml), and then dried ($MgSO_4$). Concentration of the solution under reduced pressure gave an oil (2.3 g) which was distilled, b.p. 48—50°/(0.25 mmHg) 0.333 mbar.

(c) Preparation of 1-(2-cyclooctenylmethyl)imidazole

2-Cyclooctene-1-bromomethane (0.7 g, 0.0034 mol) was added dropwise to a boiling solution of

imidazole (0.24 g, 0.0035 mol) and potassium *t*-butoxide (0.39 g, 0.0035 mol) in dry *n*-butanol, under dry nitrogen. After the addition, the reaction mixture was stirred and heated under reflux for 1 h. The pure product was obtained as described in Example 4, b.p. 108—110°/(0.02 mmHg) 0.0267 mbar.

Example 6

*Preparation of 1-(4-methylcyclohexylmethyl)imidazole*

1-Bromomethyl-4-methylcyclohexane (3.1 g, 0.0162 mol) was added dropwise to a stirred, boiling solution of imidazole (1.12 g, 0.0165 mol) and potassium *t*-butoxide (1.85 g, 0.0165 mol) in dry *n*-butanol, under dry nitrogen, After the addition, the reaction mixture was stirred and heated under reflux for 10 h.

After cooling, the reaction mixture was filtered, and then concentrated under reduced pressure. The residue was dissolved in 2*M*-hydrochloric acid (100 ml) and the solution was washed with ether (50 ml). The acid solution was basified with 10*M*-sodium hydroxide solution and extracted with chloroform (3 x 50 ml). The combined chloroform extracts were dried ($MgSO_4$) and concentrated under reduced pressure. The oily residue was purified using a silica gel column and elution with ethyl acetate/methanol (9:1). The fractions containing 1-(4-methylcyclohexylmethyl)imidazole were combined, concentrated, and the resulting oil distilled, b.p. 80°/(0.125 mmHg) 0.1667 mbar.

Example 7

*Biological Results*

Horse platelets were prepared from whole horse blood by differential centrifugation. Approximately $10^6$ platelets were homogenised in 1 ml 100 mM Tris buffer pH 7.4. Various concentrations of active compound were added and the reaction sets incubated for 5 minutes at ambient temperature. To each tube was added 20 nM of arachidonic acid containing $10^6$ DPM of labelled arachidonic acid and the tubes incubated for 3 minutes at 37°C in a shaking water bath. After incubation the radioactive products were extracted from the acidified aqueous phase with ethyl acetate and after concentration resolved by thin layer chromatography on silica gel with chloroform/methanol/acetic acid/water (90:8:1:0.8) as a developing solvent. The amount of thromboxane produced was measured by scraping the radioactive zone corresponding to thromboxane $B_2$ and estimating the radioactivity in a liquid scintillation counter.

The concentration of active compound to reduce the enzyme activity by 50% ($ED_{50}$) was established. The results are shown in Table A.

The selectivity of the active compounds was measured in a similar manner to that described above and the amount of PGE, PGF and PGD produced was determined. The greater the selectivity, the more of the prostaglandins are produced indicating lower inhibition of cyclo-oxygenase.

The $ED_{50}$ and Selectivity results are shown in Table A in which 0 indicates no selectivity; + low selectivity; ++ medium selectivity; and +++ high selectivity; and ++++ exceptionally high selectivity.

TABLE A

| Compound (Reference Compound) | $ED_{50}$ $\mu g/ml$ | Selectivity |
|---|---|---|
| (Imidazole) | ⩾500 | 0 to + |
| (1-Methylimidazole) | ⩾200 | ++ |
| 1-Cyclopentylmethylimidazole | ~5 | +++ |
| 1-Cyclohexylethylimidazole | 4 | +++ |
| 1-Cyclooctylmethylimidazole | 4 | +++ |
| 1-Cyclohex-3-enylmethylimidazole | ~5 | +++ |
| 1-Cyclobutylmethylimidazole | 50 | +++ |
| 1-Cyclopentylmethylimidazole | 10.5 | +++ |
| 1-Cycloheptylmethylimidazole | 4.7 | ++++ |
| 1-(4-Methylcyclohexylmethyl)imidazole | 6.6 | +++ |

### Example 8

Tablet formulation

| | |
|---|---|
| 1-Cyclooctylmethylimidazole (as a salt) | 150 mg |
| Starch | 25 mg |
| Polyvinylpyrrolidone | 2 mg |
| Magnesium stearate | 3 mg |

The imidazole salt is ground to a fine powder, blended with the starch and then the mixture granulated with an aqueous solution of the polyvinyl-pyrrolidone. The granules are sieved 1000 $\mu$, dried, sieved again and the magnesium stearate added. The mixture is then compressed into tablets.

In the same manner, tablets of 1-cyclohex-3-enylmethylimidazole and 1-cyclohexylethylimidazole are prepared.

### Example 9

Tablet formulation

Tablets (150 mg) of the imidazoles described in the preceding example are prepared as in the same manner from the following ingredients:

| | |
|---|---|
| The Imidazole Compound (as a salt) | 150 mg |
| Lactose | 100 mg |
| Starch | 30 mg |
| Polyvinylpyrrolidone | 2 mg |
| Magnesium stearate | 3 mg |

In the preparation, the lactose is blended with the starch.

### Example 10

Tablet formulation

Tablets (100 mg) of the imidazoles of Example 8 are prepared in the same manner from the following ingredients:

| | |
|---|---|
| The Imidazole Compound (as a salt) | 100 mg |
| Sodium starch glycolate | 10 mg |
| Polyvinylpyrrolidone | 2 mg |
| Magnesium stearate | 3 mg |

### Example 11

Tablet formulation

Tablets (150 mg) of the imidazoles of Example 8 are prepared in the same manner from the following ingredients, except that the starch, pregelled starch and imidazole compound are all blended together prior to granulation:

| | |
|---|---|
| The Imidazole Compound (as a salt) | 150 mg |
| Starch | 25 mg |
| Pregelled starch | 5 mg |
| Magnesium stearate | 3 mg |

Example 12

Injectable formulation

| | |
|---|---|
| Imidazole compound of formula (I) | 15.0 g |
| Lactic Acid B.P. | q.s. to pH 3.0 |
| Water for Injections B.P. | to 100.0 ml |

Suspend the compound in $\frac{3}{4}$ of the available quantity of water. Add sufficient Lactic Acid to dissolve the compound and to reduce the pH to 3.0. Dilute to volume with Water for Injections

Sterilise the solution by passage through a membrane filter, pore size 0.22 $\mu$m.

Distribute the solution using aseptic precautions into sterilised ampoules, 1 ml per ampoule. Seal by fusion of the glass.

Each 1 ml ampoule supplies 150 mg of the imidazole compound: 1-cyclooctylmethylimidazole fumarate.

Example 13

Injectable formulation

| | |
|---|---|
| Imidazole compound of formula (I) | 15.0 g |
| Citric Acid B.P. | q.s. to pH 3.0 |
| Chlorocresol | 0.1 g |
| Water for Injections to | 100.0 ml |

Suspend the compound in $\frac{1}{2}$ the final volume of Water for Injections. Add sufficient Citric Acid as a 10% solution in Water for Injections to dissolve the compound and reduce the pH to 3.0. Dilute to volume with Water for Injections.

Sterilise the solution by passage through a membrane filter, pore size 0.22 $\mu$m.

Distribute the solution with aseptic precautions into sterilised vials, 25 ml per vial. Stopper with sterile rubber closures and seal with an aluminium cap.

Each 1 ml of solution provides 150 mg of the compound: 1-cyclooctylmethylimidazole fumarate.

Example 14

Injectable formulation

In the manner described in the preceding two Examples, injectable formulations of 1-cyclohexylethylimidazole and 1-cyclohex-3-enylmethylimidazole salts were prepared.

Example 15

By the method described in Example 1 above the following compounds were prepared:—

(a) 1-(cyclooctylvinyl)imidazole
(b) 1-(2-cyclooctylethyl)imidazole
(c) 1-(1-cyclooctylethyl)imidazole
(d) 1-(3-cyclooctylpropyl)imidazole
(e) 1-(3-methylcyclohept-2-enylmethyl)imidazole
(f) 1-(3-methylcycloheptylmethyl)imidazole
(g) 1-(cyclohex-3-enylmethyl)imidazole m.p. 36—37°C
(h) 1-(cyclohexylethyl)imidazole b.p. 95—96°C/(0.2 mm Hg) 0.2666 mbar
(i) 1-(cyclobutylmethyl)imidazole b.p. 148—150°C/(25 mm Hg) 33.33 mbar
(j) 1-(cyclononylmethyl)imidazole

Example 16

Salts of 1-Cyclooctylmethylimidazole

A. Hydrogen Fumarate

A solution of fumaric acid (0.22 g) in hot ethanol (10 ml) was added to a solution of 1-cyclooctylmethylimidazole (0.38 g) in ethanol (4 ml). After boiling for 10 minutes the solution was evaporated to afford a white solid. Recrystallisation from ethyl acetate afforded 1-cyclooctylmethylimidazole hydrogen fumarate (0.42 g) as white needles, m.p. 147—148°.

B. Hydrogen Succinate

A solution of succinic acid (0.23 g) in ethanol (~5 ml) was added to a solution of 1-cyclooctylmethylimidazole (0.38 g) in ethanol (5 ml). Evaporation of the solution afforded a white solid. Recrystallisation of the solid from ethyl acetate afforded 1-cyclooctylmethylimidazole hydrogen succinate (0.27 g) as colourless plates, m.p. 86—87°.

C. Oxalate

A solution of oxalic acid (0.17 g) and 1-cyclooctylmethylimidazole (0.38 g) in ethanol (20 ml) was boiled for 0.25 h, when evaporated of the solution afforded a white solid. Recrystallisation of the solid from ethyl acetate/ethanol/petroleum ether (b.p. 40—60°) afforded 1-cyclooctylmethylimidazole oxalate as white needles, m.p. 141—142°.

D. Hydrochloride

1-Cyclooctylmethylimidazole (~0.3 g) was dissolved in dry ether (30 ml), when a stream of dry hydrogen chloride was passed through the solution at —20°. The resulting white precipitate was filtered off under dry nitrogen and recrystallised from ethyl acetate/petroleum ether (b.p. 40—60°) to afford 1-cyclooctylmethylimidazole hydrochloride as a white solid, m.p. 20—22°C.

## Claims

1. Compounds of the general formula

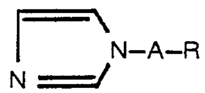

(I)

in which A is a straight or branched, saturated or unsaturated acyclic hydrocarbon radical of from 1 to 3 carbon atoms and R is a cycloalkyl or cycloalkenyl group of from 4 to 9 carbon atoms optionally substituted by one or more alkyl groups each containing from 1 to 4 carbon atoms, with the proviso that when A is a methylene radical, R is not unsubstituted cyclohexyl, the compound being the free base or a pharmaceutically acceptable acid addition salt thereof.

2. Compounds as defined in claim 1 characterised in that A is $—CH_2—$ or $—(CH_2)_2—$.

3. Compounds as defined in claim 2 characterised in that R is cyclopentyl, cyclohexyl, cyclooctyl or cyclohexenyl.

4. A compound selected from 1-cyclohex-3-enylmethylimidazole and 1-cycloheptylmethylimidazole, and pharmaceutically acceptable acid addition salts thereof.

5. 1-Cyclooctylmethylimidazole or a pharmaceutically acceptable acid addition salt thereof.

6. 1-Cyclohexylethylimidazole or a pharmaceutically acceptable addition salt thereof.

7. A method of preparing a compound or a pharmaceutically acceptable acid addition salt thereof as defined in claim 1 characterised by reacting in a method known per se imidazole or a salt thereof with an alkylating agent of the formula Z—A—R wherein A and R are as defined in claim 1 and Z is a leaving group.

8. A method of preparing a compound of formula (I) as defined in claim 1 or a pharmaceutically acceptable acid addition salt thereof characterised by desulphurising or dehalogenating in a method known per se a substituted imidazole of the formula

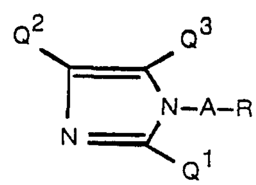

wherein A and R are defined in formula (I) and $Q^1$, $Q^2$ and $Q^3$ are the same or different, at least one being a radical selected from thio(—SH), $C_{1-4}$ alkylthio and halo, the other being a radical having the same function or is hydrogen.

9. A method of preparing a compound of formula (I) as defined in claim 1 or a pharmaceutically acceptable acid addition salt thereof, characterised by decarboxylating in a method known per se a compound of the formula

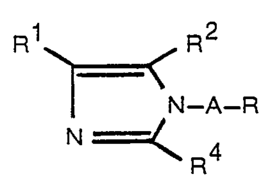

wherein A and R are defined in formula (I), at least one of $R^1$, $R^2$ and $R^4$ is carboxyl or a derivative thereof, and the others are hydrogen, carboxyl or a derivative thereof.

10. A method of preparing a compound of formula (I) as defined in claim 1 or a pharmaceutically acceptable acid addition salt thereof, characterised by reacting in a method known per se a precursor of the formula

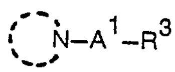 wherein 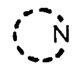

is 1-imidazoline, 1-imidazole or 1-pyrazole, $A^1$ is a straight or branched saturated or unsaturated acyclic $C_{1-3}$ hydrocarbon radical, and $R^3$ is a cycloalkyl or cycloalkenyl radical of 4 to 9 carbon atoms optionally substituted by alkyl as defined in formula (I) provided that at least one of

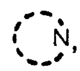,

$A^1$ and $R^3$ is other than 1-imidazole, a saturated acyclic hydrocarbon and an optionally substituted cycloalkyl group as defined in formula (I) respectively, with a reagent serving to convert said precursor into a compound of general formula (I).

**Revendications**

1. Composée de formule générale

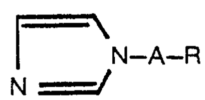

(I)

où A représente un radical hydrocarboné acyclique saturé ou insaturé en chaîne droite ou ramifiée de 1 à 3 atomes de carbone et R représente un radical cycloalcoyle ou cycloalcényle de 4 à 9 atomes de carbone éventuellement substitué par un ou plusieurs radicaux alcoyle dont chacun compte 1 à 4 atomes de carbone, avec la restriction que lorsque A est un radical méthylène, R n'est pas un radical cyclohexyle non substitué, le composé étant la base libre ou un de ses sels d'addition d'acides pharmaceutiquement acceptables.

2. Composés tels que définis dans la revendication 1, caractérisés en ce que A représente $—CH_2—$ ou $—(CH_2)_2—$.

3. Composés tels que définis dans la revendication 2, caractérisés en ce que R représente un radical cyclopentyle, cyclohexyle, cyclooctyle ou cyclohexényle.

4. Composé choisi entre le 1-cyclohex-3-énylméthylimidazole et le 1-cycloheptylméthylimidazole, outre leurs sels d'addition d'acides pharmaceutiquement acceptables.

5. Le 1-cyclooctylméthylimidazole ou un de ses sels d'addition d'acides pharmaceutiquement acceptables.

6. Le 1-cyclohexyléthylimidazole ou un de ses sels d'addition d'acides pharmaceutiquement acceptables.

7. Procédé de préparation d'un composé ou d'un sel d'addition d'acide pharmaceutiquement acceptable de celui-ci tel que défini dans la revendication 1, caractérisé en ce qu'on fait réagir suivant un procédé connu l'imidazole ou un sel de celui-ci avec un agent d'alcoylation de formule Z—A—R, où A et R sont tels que définis dans la revendication 1 et Z représente un radical labile.

8. Procédé de préparation d'un composé de formule (I) tel que défini dans la revendication 1 ou d'un sel d'addition d'acide pharmaceutiquement acceptable de celui-ci, caractérisé en ce qu'on désulfure ou déshalogène suivant un procédé connu un imidazole substitué de formule

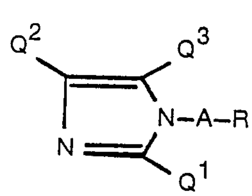

où A et R sont tels que définis dans la formule (I) et $Q^1$, $Q^2$ et $Q^3$ sont identiques ou différents, au moins l'un d'entre eux étant un radical choisi entre thio(—SH), $C_{1-4}$ alcoylthio et halogéno, l'autre étant un radical ayant la même fonction ou étant un atome d'hydrogène.

9. Procédé de préparation d'un composé de formule (I) tel que défini dans la revendication 1 ou

d'un sel d'addition d'acide pharmaceutiquement acceptable de celui-ci, caractérisé en ce qu'on décarboxyle suivant un procédé connu un composé de formule:

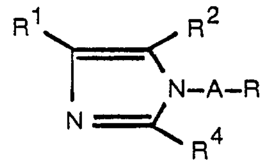

où A et R sont tels que définis dans la formule (I), au moins l'un d'entre $R^1$, $R^2$ et $R^4$ est un radical carboxyle ou un dérivé de celui-ci et les autres sont des atomes d'hydrogène ou radicaux carboxyle ou dérivés de ceux-ci.

10. Procédé de préparation d'un composé de formula (I) tel que défini dans la revendication 1 ou d'un sel d'addition d'acide pharmaceutiquement acceptable de celui-ci, caractérisé en ce qu'on fait réagir suivant un procédé connu un précurseur de formule

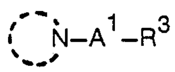 où 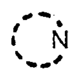

représente la 1-imidazoline, le 1-imidazole ou le 1-pyrazole, $A^1$ représente un radical hydrocarboné en $C_{1-3}$ acyclique saturé ou insaturé en chaîne droite ou ramifiée et $R^3$ représente un radical cycloalcoyle ou cycloalcényle de 4 à 9 atomes de carbone éventuellement substitué par un radical alcoyle tel que défini dans la formule (I), avec la restriction qu'au moins l'un d'entre

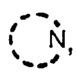,

$A^1$ et $R^3$ est autre que le 1-imidazole, un radical hydrocarboné acyclique saturé et un radical cycloalcoyle éventuellement substitué tel que défini dans la formule (I), respectivement, avec un réactif servant à convertir ce précurseur en un composé de formule générale (I).

## Patentansprüche

1. Verbindungen der allgemeinen Formel I

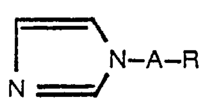

(I)

worin A einen geradkettigen oder verzweigtkettigen, gesättigten oder ungesättigten acyclischen Kohlenwasserstoffrest mit 1 bis 3 Kohlenstoffatomen bedeutet und R einen Cycloalkyl- oder Cycloalkenylrest mit 4 bis 9 Kohlenstoffatomen, der gegebenenfalls durch einen oder mehrere Alkylreste mit jeweils 1 bis 4 Kohlenstoffatomen substituiert ist, darstellt, mit der Maßgabe, daß sofern A ein Methylenrest ist, R nicht unsubstituiertes Cyclohexyl bedeutet, wobei die Verbindung die freie Base oder ein pharmazeutisch verträgliches Säureadditionssalz davon ist.

2. Verbindungen nach Anspruch 1, dadurch gekennzeichnet, daß A der Rest $—CH_2—$ oder $—(CH_2)_2—$ ist.

3. Verbindungen nach Anspruch 2, dadurch gekennzeichnet, daß R Cyclopentyl, Cyclohexyl, Cyclooctyl oder Cyclohexenyl ist.

4. Verbindung, ausgewählt unter 1-Cyclohex-3-enylmethylimidazol und 1-Cycloheptylmethylimidazol und deren pharmazeutisch verträglichen Säureadditionssalzen.

5. 1-Cyclooctylmethylimidazol oder ein pharmazeutisch verträgliches Säureadditionssalz davon.

6. 1-Cyclohexylethylimidazol oder ein pharmazeutisch verträgliches Säureadditionsalz davon.

7. Verfahren zur Herstellung einer Verbindung oder eines pharmazeutisch verträglichen Säureadditionssalzes davon gemäß der Definition in Anspruch 1, dadurch gekennzeichnet, daß in einem an sich bekannten Verfahren Imidazol oder ein Salz davon mit einem Alkylierungsmittel der Formel Z—A—R, worin A und R die in Anspruch 1 angegebene Bedeutung haben, und Z eine Abspaltgruppe ist, umgesetzt wird.

8. Verfahren zur Herstellung einer Verbindung nach Formel I gemäß der Definition in Anspruch 1 oder eines pharmazeutisch verträglichen Säureadditionssalzes davon, gekennzeichnet durch Desulfurierung oder Dehalogenierung in einem an sich bekannten Verfahren eines substituierten Imidazols der Formel

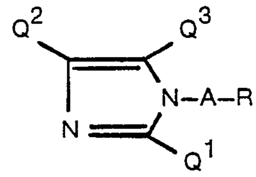

worin A und R die in Formel I angegebene Bedeutung haben und $Q^1$, $Q^2$ und $Q^3$ gleich oder unterschiedlich sind, wobei mindestens einer dieser Reste ein Thio(—SH)-, $C_{1-4}$-Alkylthio- oder Halogenrest ist, and wobei der andere Rest dieselbe Funktion hat oder Wasserstoff ist.

9. Verfahren zur Herstellung einer Verbindung nach Formel I gemäß der Definition in Anspruch 1 oder eines pharmazeutisch verträglichen Säureadditionssalzes davon, gekennzeichnet durch Decarboxylierung in einem an sich bekannten Verfahren einer Verbindung der Formel

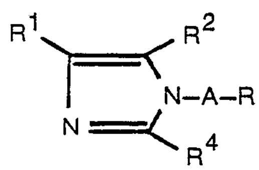

worin A und R die in Formel I genannte Bedeutung haben, wobei mindestens einer der Reste $R^1$, $R^2$ und $R^4$ ein Carboxylrest oder ein Derivat davon ist und die anderen Reste Wasserstoff, Carboxyl oder ein Derivat davon sind.

10. Verfahren zur Herstellung einer Verbindung der Formel I gemäß der Definition in Anspruch 1 oder eines pharmazeutisch verträglichen Säureadditionssalzes davon, gekennzeichnet durch die Umsetzung in einem an sich bekannten Verfahren einer Vorstufen-Verbindung der Formel

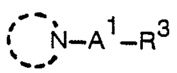 worin 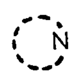

1-Imidazolin, 1-Imidazol oder 1-Pyrazol ist, $A^1$ einen geradkettigen oder verzweigtkettigen, gesättigten oder ungesättigten acyclischen $C_{1-3}$-Kohlenwasserstoffrest bedeutet und $R^3$ einen Cycloalkyl- oder Cycloalkenylrest mit 4 bis 9 Kohlenstoffatomen, der gegebenenfalls durch Alkyl gemäß der Definition in Formel I substituiert ist, darstellt, mit der Maßgabe, daß mindestens einer der Reste

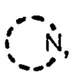,

$A^1$ und $R^3$ etwas anderes als 1-Imidazol, ein gesättigter acyclischer Kohlenwasserstoffrest bzw. ein gegebenenfalls substituierter Cycloalkylrest gemäß der Definition in Formel I ist, mit einem Reagens, welches dazu dient, die Vorstufen-Verbindung in eine Verbindung der allgemeinen Formel I zu überführen.